# EUROPEAN PATENT SPECIFICATION

(11) **EP 2 947 075 B1**
(45) Date of publication and mention of the grant of the patent: **16.11.2016**
(21) Application number: 13874792.8
(22) Date of filing: 05.02.2013
(51) Int. Cl.: C07D 285/13

(54) **SYNTHESIS METHOD OF THIADIAZOLYLAMIDE DERIVATIVE**
VERFAHREN ZUR SYNTHESE EINES THIADIAZOLYLAMIDDERIVATS
PROCÉDÉ DE SYNTHÈSE DE DÉRIVÉ DE THIADIAZOLYLAMIDE

(43) Date of publication of application: 25.11.2015
(73) Proprietor: Zhejiang Zhuji United Chemicals Co., Ltd, Zhuji, Zhejiang 311800 (CN)
(72) Inventor: SU, Yehua, Zhuji Zhejiang 311800 (CN); CAI, Guoping, Zhuji Zhejiang 311800 (CN); YANG, Zhenghe, Zhuji Zhejiang 311800 (CN); CHEN, Bangchi, Zhuji Zhejiang 311800 (CN)
(74) Representative: Rogers, Alex Lee
(86) International application number: PCT/CN2013/071394
(87) International publication number: WO 2014/121439

(56) References cited:
- CN-A- 1 346 596
- CN-A- 1 361 772
- CN-A- 1 384 828

## Description

### Technical field of the invention

This invention belongs to the field of organic synthesis of thiadiazolylamides. Specifically, this invention is related to a synthesis method for thiadiazolylamide derivatives.

### Background of the invention

US4708731, US4645525 andUS4585471 reported that thiadiazolylamide derivatives possess herbicidal activities. In 1983, US4645525 disclosed a synthetic method for N-methyl-2-[(5-(trifluoromethyl)-1,3,4-thiadiazoly-2-yl)-oxy]-N-acetyl aniline. This method involved: reacting N-methyl-2-hydroxy-N-acetyl aniline with calcium oxide in dimethylsulfoxide at 50 °C for 1 hour, adding 5-bromo-2-trifluoromethyl-1,3,4-thiadiazole to the reaction mixture, and stirring the reaction mixture at 50 °C for 40 hours. The mixture was poured into water. The oily material precipitated was extracted with methylene chloride. The solvents were removed to give final product. The yield was about 90%. This preparation method used bromine as leaving group, but the raw material 5-bromo-2-trifluoromethyl-1,3,4-thiadiazole used in this method was difficult to prepare, the reaction time was long, the solvent dimethylsulfoxide was difficult to recover.

In 1984, US 4708731 disclosed that a class of [(5-chloroalkyl-1,3,4-thiadiazolyl-2-yl 1,3,4-thiadiazolyl-2-yl 1,3,4-thiadiazolyl-2-yl)-oxy]acetamides possess herbicidal activity. This type of thiadiazole acetamides were prepared by reaction of 2-chloro-1,3,4-thiadiazole and hydroxy-N-acetyl aniline in toluene. US4708731 disclosed a synthesis method for preparing N-ethyl-2-[(5-(trichloromethyl)-1,3,4-thiadiazol- 2-yl)-oxy]-N-acetylethylamine. This method comprised dropwise addition of aqueous solution of sodium hydroxide to a toluene solution of 2-chloro-5-trichloromethyl-1,3,4-thiadiazole and N-ethyl-2-hydroxy-N-acetylethylamine at -10 °C. After the addition was completed, the reaction was continued for 12 hours at 0-5 °C. The toluene phase was separated and washed with water until the pH was neutral. The solvent was removed under vaccum, the residue was slurried with petroleum ether to give a solid. Filtration, drying gave the final product. The yield was about 42%. In this preparation method, chlorine was used to replace bromine as the leaving group. The cost of starting materials was lower than the 5-bromo-2-trifluoromethyl-1,3,4-thiadiazole process, however the reaction time was long, the final reaction yield was only 42%.

In 1986, US4585471 disclosed a method for preparing 2-[(5-(trifluoromethyl)-1,3,4-thiadiazolyl-2-yl)-oxy]acetylamino-2-ethyl piperidine. This synthesis method comprised reaction of N-(hydroxyacetyl)-2-ethylpiperidine and 2-chloro-5-trifluoromethyl-1,3,4-thiadiazole in the presence of sodium tert-butoxide in tert-butanol for 3 hours. The reaction temperature was 20-30 °C. Dichloromethane was added for extraction, the mixture was acidified with dilute hydrochloric acid, washed with water. Removal of solvent gave the target product. The yield was about 66%. This preparation method used more expensive sodium tert-butoxide, the yield was increased to some degree, but still not satisfactory.

In 1990, US4968342 disclosed a synthesis method for 3'-chloro-N-(isopropyl)-2-[(5-trifluoromethyl-1,3,4-thiadiazolyl-2-yl1,3,4-thiadiazolyl-2-yl1,3,4-thiadiazolyl-2-yl)-oxy]acetanilide. This synthesis method comprised of dissolving 2-(methylsulphonyl)-5-(trifluoromethyl)-1,3,4-thiadiazole and 3'-chloro-N-(isopropyl)-2-hydroxyacetanilide in acetone, dropwise addition of an aqueous solution of sodium hydroxide at -20 °C. The reaction was carried out for 3 hours at -20 °C, the reaction mixture was poured into water, the crystallized product was obtained by filtration. The yield was about 85%. Compared to previous processes, the yield of this preparation method was improved to some degree (about 85%), but this process used water miscible solvent, making the starting material 2-(methylsulphonyl)-5-(trifluoromethyl)-1,3,4-thiadiazole to hydrolyze readily. The reaction had to be carried out at low temperature, and recycling of acetone was difficult.

In 1997, US5895818 and US5792872 disclosed a synthesis method for 4'-fluoro-N-isopropyl-2-[(5-trifluoromethyl-1,3,4-thidiazol-2-yl)-oxy] acetanilide. According to this method, 2-(methylsulfonyl)- 5-trifluoromethyl-1,3,4-thiadiazole and 4'-fluoro-N-isopropyl-2-hydroxyacetanilide were dissolved in toluene. A solution of sodium hydroxide was added dropwise at 0-5 °C. After the addition was completed, the reaction was continued for 2 hours at 5-10 °C. The reaction mixture was acidified with dilute hydrochloric acid to a pH of 5.0. The toluene phase was separated, the aqueous phase was extracted with toluene. The toluene phases were combined. Removal of solvent gave the target product, the yield was more than 93%. This synthesis method used methylsulfonyl as a leaving group. The reaction conditions were relatively mild, the yield was relatively high. However methylsulfonyl group needed to be obtained by oxidation of methylthio group, in which the oxidation state of the sulfur atom was increased from -2 to +2, requiring to obtain two oxygen atoms from the oxidizing reagent. Hence, more than two equivalents of oxidizing reagent were needed, the atomic efficiency was poor. At the same time, the preparation of the starting material methylsulfonyl-thiadiazole used highly concentrated hydrogen peroxide (US5856499). Use of highly concentrated hydrogen peroxide in large scale production is a major safety concern.

CN1361772 relates to substituted heteroaryloxyacetanilides and their use as herbicides.

CN1346596 relates to herbicidal heterocyclic oxyacetyl substituted phenylamine compounds, and preparing process and herbicide composition thereof.

### Detailed description of the invention

The existing technology for the preparation of thiadiazoleacetamides suffer from many problems, such as difficulties in preparing raw materials, high costs, low yields, long reaction times, low atomic efficiency, use of highly dangerous reagents, and etc. Therefore, there is urgent need to develop economical, safe and simple method for synthesizing thiadiazolylamides.

The present invention provides a method for preparing thiadiazolylamide compound of the formula (I): 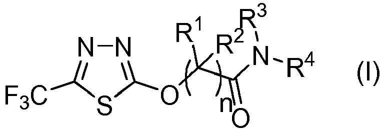 wherein
R¹, R², R³, R⁴ are H, C₁-C₆- alkyl, or C₆-C₁₄ aryl, ,
n is 1-6,
comprising reacting the compound of the formula (II): 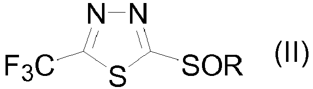 wherein
R is C₁-C₆- alkyl, or C₆-C₁₄ aryl,
with the compound of the formula (III): 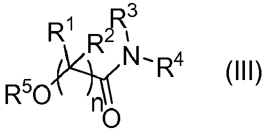 wherein
R¹, R², R³, R⁴ are defined as above,
n is 1-6,
R⁵ is H, C₁-C₄- alkyl acyl, C₆-C₁₄ aryl acyl,
in a solvent and in the presence of a base. The reaction can also be performed in the presence of a catalyst.

The synthesis method of the present invention comprises the following step: dissolving 2-alkylsulphinyl-5-trifluoromethyl-1,3,4-thiadiazole (II) and amide (III) in a water immiscible solvent, and reacting in the presence of a base, separating organic phase after the reaction is completed, washing with water, removing solvent to give 2-[(5-(trifluoromethyl)-1,3,4-thiadiazolyl-2-yl)-oxy]amide (I). The reaction can be also performed in the presence of a catalyst.

R in compound (II) is C₁-C₆- alkyl, C₆-C₁₄ aryl, preferably is C₁-C₆-alkyl, more preferably is methyl.

R¹, R², R³, R⁴ in compound (III) are H, C₁-C₆- alkyl, or C₆-C₁₄ aryl, R¹ and R² preferably are H or C₁-C₆- alkyl, more preferably are H. R³ preferably is H or C₁-C₆- alkyl, more preferably is C₁-C₆- alkyl, most preferably is isopropyl. R⁴ preferably is C₆-C₁₄ aryl, more preferably is C₆-C₁₄ aryl, most preferably is 4-fluorophenyl. n is 1-6, preferably is 1-3, most preferably is 1.

Optionally, the water-immiscible organic solvent is n-hexane, petroleum ether, dichloromethane, xylene or toluene.

Optionally, the base is organic or inorganic base, preferably is inorganic base, more preferably is alkali metal hydroxide, alkali metal carbonate, alkaline earth metal hydroxide or alkaline earth metal carbonate, most preferably is lithium hydroxide, sodium hydroxide or potassium hydroxide.

Optionally, the catalyst is quaternary ammonium salt, quaternary phosphorus salt, crown ether or polyether, preferably is quaternary ammonium salt, more preferably is tetrabutylammonium halide, most preferably is tetrabutylammonium bromide.

The starting material in the present invention 2-alkylsulphinyl-5-trifluoromethyl-1,3,4-thiadiazole (II) can be prepared by various oxidation methods. The synthesis protocol disclosed in the prior art US6437189 can be referred to. The other starting material amide (III) can also be synthesized by a number of methods. The synthesis protocol in the prior art US5631403 and US4334073 can be referred to.

The reaction of 2-alkylsulphinyl-5-trifluoromethyl-1,3,4-thiadiazole (II) and amide (III) is more suitably carried out at a relatively low temperature. Preferred reaction temperature is -20°C to 25°C, more preferred temperature is -10°C to 15°C, most preferred temperature is 0°C to 10°C. The molar ratio of the solvent used in the reaction to the compound (II) or (III) preferably is 5:1 to 60:1, more preferred ratio is 5:1 to 30:1, most preferred ratio is 10:1 to 20:1.

In the embodiment of the present invention, the weight percent of base is 10 % to 100%, preferably is 15 % - 50 %. The molar ratio of the base used to the starting compound (II) is preferably 1:1 to 1:2, more preferred molar ratio is 1:1 to 1:1.5.

The invention used 2-sulphinyl-5-trifluoromethyl-1,3,4-thiadiazole (II) and amide (III) as starting materials for synthesis of 2-[(5-(trifluoromethyl)-1,3,4-thiadiazolyl-2-yl)-oxy]amide (I). It has many advantages, mainly shown in high atomic efficiency, simple raw material preparation, simple reaction manipulation, high reaction speed, high yield, producing few wastes, amenable for industrial production.

### Explanation of the attached figures

Fig.1 is the 1H-NMR spectrum of 4'-fluoro-N-(isopropyl)-2-[(5-(trifluoromethyl)-1,3,4-thidiazol-2-yl)oxy]acetanilide.
Fig.2 is the high resolution mass spectrum (ESI+) of 4'-fluoro-N-(isopropyl)-2-[(5-(trifluoromethyl)-1,3,4-thidiazol-2-yl)oxy]acetanilide.

### Examples

The following examples provide further illustration for the present invention along with the attached figures.

### Example 1 Synthesis of 4'-fluoro-N-(isopropyl)-2-[(5-trifluoromethyl-1,3,4-thidiazol-2-yl)oxy]acetanilide

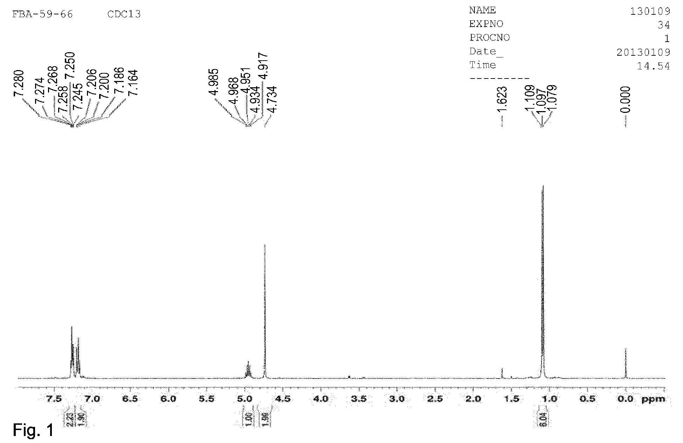

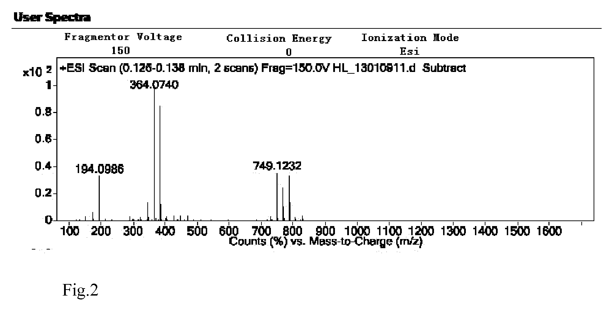

To a 500 mL three-necked round bottom flask equipped with mechanical stirrer, thermometer and 50 mL constant pressurized addition funnel was charged with 21.9 g (0.1 mol) of 2-methylsulphinyl-5-trifluoromethyl-1,3,4-thiadiazole and 21.5 g (0.1 mol) of 4'-fluoro-N-isopropyl-2-hydroxyacetanilide. 100 mL of toluene was added. Cooling to below 5°C with ice water bath, an aqueous solution containing 4 g (0.1 mol) of sodium hydroxide was added dropwise to the reaction mixture with stirring. After the addition was completed, the reaction was continually stirred for 1 hour. The toluene phase was separated, washed with water twice. The toluene was removed under reduced pressure to provide 36 g of white solid. The yield was 96%. ¹H NMR(CDCl₃) δ7.28(m, 2H), 7.20(m, 2H), 4.95(m, 1H), 4.73(s, 1H), 1.10(s, 3H), 1.08(s, 3H), as shown in Fig.1. High resolution mass spectrum (ESI⁺) m/z 364.0740 [C₁₄H₁₃F₄N₃O₂S+H requires 364.0737], as shown in Fig.2.

Example 2 Synthesis of 4'-fluoro-N-(isopropyl)-2-[(5-trifluoromethyl-1,3,4-thidiazol-2-yl)oxy]acetanilide

To a 1000 mL three-necked round bottom flask equipped with mechanical stirrer, thermometer and 50 mL constant pressurized addition funnel was charged with 21.9 g (0.1 mol) of 2-methylsulphinyl-5-trifluoromethyl-1,3,4-thiadiazole and 21.5 g (0.1 mol) of 4'-fluoro-N-isopropyl-2-hydroxyacetanilide. 0.1g of tetrabutylammonium bromide and 100 mL of toluene were added. Cooling to below 5°C with ice water bath, an aqueous solution containing 12 g (0.3 mol) of sodium hydroxide was added portion-wise to the reaction mixture with stirring. After the addition was completed, the reaction was continually stirred for 5 hours. The toluene phase was separated, washed with water twice. The toluene was removed under reduced pressure to provide 35.4 g of white solid. The yield was 91.8%.

### Example 3 Synthesis of 4'-fluoro-N-(isopropyl)-2-[(5-trifluoromethyl-1,3,4-thidiazol-2-yl)oxy]acetanilide

To a 500 mL three-necked round bottom flask equipped with mechanical stirrer, thermometer and 50 mL constant pressurized addition funnel was charged with 21.9 g (0.1 mol) of 2-methylsulphinyl-5-trifluoromethyl-1,3,4-thiadiazole and 21.5 g (0.1 mol) of 4'-fluoro-N-isopropyl-2-hydroxyacetanilide. 200 mL of dichloromethane was added. Cooling to below 5°C with ice water bath, an aqueous solution containing 4.4 g (0.11 mol) of sodium hydroxide was added dropwise to the reaction mixture with stirring. After the addition was completed, the reaction was continually stirred for 2 hours. The organic phase was separated, washed with water twice. The solvent was removed under reduced pressure to provide 36.5 g of white solid. The yield was 98.5%.

### Example 4 Synthesis of 4'-fluoro-N-(isopropyl)-2-[(5-trifluoromethyl-1,3,4-thidiazol-2-yl)oxy]acetanilide

To a 500 mL three-necked round bottom flask equipped with mechanical stirrer, thermometer and 50 mL constant pressurized addition funnel was charged with 21.9 g (0.1 mol) of 2-methylsulphinyl-5-trifluoromethyl-1,3,4-thiadiazole and 21.5 g (0.1 mol) of 4'-fluoro-N-isopropyl-2-hydroxyacetanilide. 200 mL of dichloromethane was added. Cooling to below 5°C with ice water bath, an aqueous solution containing 4.4 g (0.11 mol) of sodium hydroxide was added dropwise to the reaction mixture with stirring. After the addition was completed, the reaction was continually stirred for 2 hours. The organic phase was separated, washed with water twice. The solvent was removed under reduced pressure to provide 35.3 g of white solid. The yield was 93.5%.

### Example 5 Synthesis of

### 4'-fluoro-N-(isopropyl)-2-[(5-trifluoromethyl-1,3,4-thidiazol-2-yl)oxy]acetanilide

To a three-necked round bottom flask equipped with mechanical stirrer, thermometer and constant pressurized addition funnel was charged with 2-methylsulphinyl-5-trifluoromethyl-1,3,4-thiadiazole and 4'-fluoro-N-isopropyl-2-hydroxyacetanilide. Toluene was added. Cooling to below 5°C with ice water bath, an aqueous solution containing sodium hydroxide was added dropwise to the reaction mixture with stirring. After the addition was completed, the reaction was continually stirred for 3 hours. The toluene phase was separated, washed with water twice. The toluene was removed under reduced pressure to provide the target product.

Although the invention has been described in detail in the foregoing for the purpose of illustration, it is to be understood that such detail is solely for that purpose, and should not be considered as limitation to the present invention. Variations made therein by those skilled in the art under the revelation and instruction of the present invention should all fall into the protection scope of the present invention.

## Claims

1. A synthesis method for thiadiazolylamide compound of the formula (I): 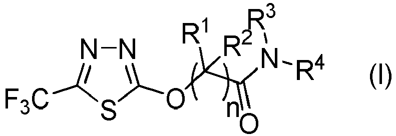 wherein R¹, R², R³, R⁴ are H, C₁-C₆ alkyl, or C₆-C₁₄ aryl, n is 1-6,
comprising reacting the compound of the formula (II): 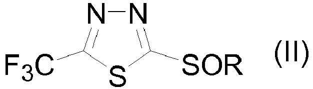 wherein R is C₁-C₆ alkyl, or C₆-C₁₄ aryl, with the compound of the formula (III): 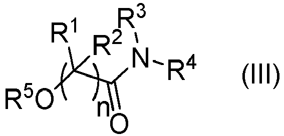 wherein
R¹, R², R³, R⁴ are defined as above,
n is 1-6,
R⁵ is H, C₁-C₄ alkyl acyl, or C₆-C₁₄ aryl acyl,
in a solvent and in the presence of a base.

2. The process according to claim 1, wherein R in compound (II) is methyl.

3. The process according to claim 1, wherein R¹, R² are H or C₁-C₆ alkyl.

4. The process according to claim 1, wherein R³ is C₁-C₆ alkyl.

5. The process according to claim 1, wherein R⁴ is C₆-C₁₄ aryl

6. The process according to claim 1, wherein R¹, R² are H, R³ is isopropyl, R⁴ is 4-fluorophenyl.

7. The process according to claim 1, wherein R⁵ is H.

8. The process according to claim 1, wherein n in compound (III) is 1.

9. The process according to claim 1, wherein the solvent is n-hexane, petroleum ether, dichloromethane, xylene or toluene.

10. The process according to claim 1, wherein the base is lithium hydroxide, sodium hydroxide or potassium hydroxide.

11. The process according to claim 1, wherein the reaction is performed in the presence of a catalyst.

12. The process according to claim 11, wherein the catalyst is tetrabutylammonium bromide.

## Patentansprüche

1. Syntheseverfahren für eine Thiadiazolylamidverbindung der Formel (I): 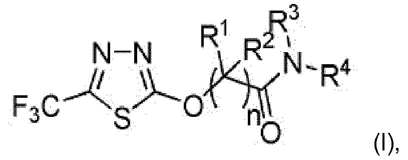 worin R¹, R², R³, R⁴ H, C₁-C₆-Alkyl oder C₆-C₁₄-Aryl sind, n 1 bis 6 ist,
umfassend Reagieren der Verbindung der Formel (II) 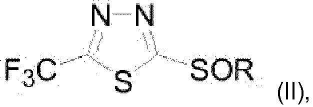 worin R C₁-C₆-Alkyl oder C₆-C₁₄-Aryl ist, mit der Verbindung der Formel (III) 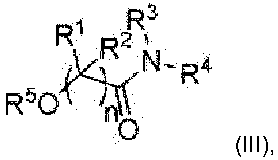 worin
R¹, R², R³, R⁴ wie oben definiert sind,
n 1 bis 6 ist,
R⁵ H, C₁-C₄-Alkylacyl oder C₆-C₁₄-Arylacyl ist,
in einem Lösungsmittel und in der Anwesenheit einer Lauge.

2. Verfahren gemäß Anspruch 1, wobei R in Verbindung (II) Methyl ist.

3. Verfahren gemäß Anspruch 1, wobei R¹, R² H oder C₁-C₆-Alkyl sind.

4. Verfahren gemäß Anspruch 1, wobei R³ C₁-C₆-Alkyl ist.

5. Verfahren gemäß Anspruch 1, wobei R^{a} C₆-C₁₄-Aryl.

6. Verfahren gemäß Anspruch 1, wobei R¹, R² H sind, R³ Isopropyl ist und R⁴ 4-Fluorophenyl ist.

7. Verfahren gemäß Anspruch 1, wobei R⁵ H ist.

8. Verfahren gemäß Anspruch 1, wobei n in Verbindung (III) 1 ist.

9. Verfahren gemäß Anspruch 1, wobei das Lösungsmittel n-Hexan, Petroleumether, Dichloromethan, Xylol oder Toluol ist.

10. Verfahren gemäß Anspruch 1, wobei die Lauge Lithiumhydroxyd, Natriumhydroxyd oder Kaliumhydroxyd ist.

11. Verfahren gemäß Anspruch 1, wobei die Reaktion in der Anwesenheit eines Katalysators ausgeführt wird.

12. Verfahren gemäß Anspruch 11, wobei der Katalysator Tetrabutylammoniumbromid ist.

## Revendications

1. Procédé de synthèse pour un composé thiadiazolylamide de la fromule (I) : 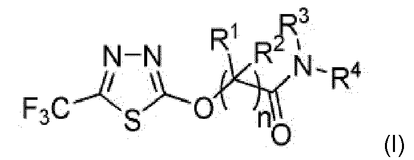 dans lequel R¹, R², R³, R⁴ sont H, C₁-C₆-alkyle, ou C₆-C₁₄-aryle, n est 1 à 6,
comprenant la réaction du composé de la formule (II) : 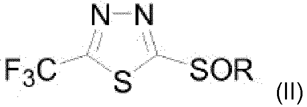 dans lequel R est C₁-C₆-alkyle, ou C₆-C₁₄-aryle, avec le composé de la formule (III) : 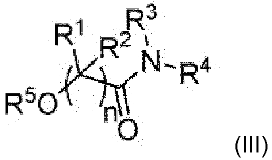 dans lequel
R¹, R², R³, R⁴ sont définis comme ci-dessus,
n est 1 à 6,
R⁵ est H, C₁-C₄-alkylacyle ou C₆-C₁₄-arylacyle,
dans un solvant et dans la présence d'une base.

2. Procédé selon la revendication 1, dans lequel R dans le composé (II) est méthyle.

3. Procédé selon la revendication 1, dans lequel R¹, R² sont H ou C₁-C₆-alkyle.

4. Procédé selon la revendication 1, dans lequel R³ est C₁-C₆-alkyle.

5. Procédé selon la revendication 1, dans lequel R⁴ est C₆-C₁₄-aryle.

6. Procédé selon la revendication 1, dans lequel R¹, R² sont H, R³ est l'isopropyle, R⁴ est le 4-fluorophényle.

7. Procédé selon la revendication 1, dans lequel R⁵ est H.

8. Procédé selon la revendication 1, dans lequel n dans le composé (III) est 1.

9. Procédé selon la revendication 1, dans lequel le solvant est le n-hexane, l'éther de pétrole, le dichlorométhane, le xylène ou le toluène.

10. Procédé selon la revendication 1, dans lequel la base est l'hydroxyde de lithium, l'hydroxyde de sodium ou l'hydroxyde de potassium.

11. Procédé selon la revendication 1, dans lequel la réaction se déroule en la présence d'un catalyseur.

12. Procédé selon la revendication 11, dans lequel le catalyseur est le bromure de tétrabutylammonium.
